# EUROPEAN PATENT SPECIFICATION

(11) **EP 2 722 034 B1**
(45) Date of publication and mention of the grant of the patent: **16.09.2020**
(21) Application number: 13189185.5
(22) Date of filing: 17.10.2013
(51) Int. Cl.: A61K 9/00, A61K 9/20, A61K 31/4184, A61K 31/4439, A61K 9/28

(54) **ORAL PHARMACEUTICAL FORMULATIONS COMPRISING DABIGATRAN**
ORALE PHARMAZEUTISCHE ZUSAMMENSETZUNGEN ENTHALTEND DABIGATRAN
FORMULATIONS PHARMACEUTIQUES ORALES COMPRENANT DU DABIGATRAN

(30) Priority: 19.10.2012 TR 201212083; 01.10.2013 TR 201311483
(43) Date of publication of application: 23.04.2014
(73) Proprietor: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Saydam, Mehtap, 34460 Istanbul (TR)
(74) Representative: Sevinç, Erkan

(56) References cited:
- WO-A1-2010/055021
- WO-A2-2006/103206
- WO-A2-2008/009638
- US-A- 6 087 380
- US-A1- 2005 038 077
- NISHIO HITOSHI ET AL: "New therapeutic option for thromboembolism--dabigatran etexilate", EXPERT OPINION ON PHARMACOTHERAPY, ASHLEY PUBLICATIONS LTD, LONDON, UK, vol. 9, no. 14, 1 October 2008 (2008-10-01), pages 2509-2517, XP009127716, ISSN: 1465-6566, DOI: 10.1517/14656566.9.14.2509
- BAETZ B E ET AL: "Dabigatran etexilate: An oral direct thrombin inhibitor for prophylaxis and treatment of thromboembolic diseases", PHARMACOTHERAPY : THE JOURNAL OF HUMAN PHARMACOLOGY AND DRUG THERAPY, BOSTON, US, vol. 28, no. 11 PART 1, 1 November 2008 (2008-11-01), pages 1354-1373, XP008100341, ISSN: 0277-0008

## Description

### Technical Field

The present invention relates to a novel pharmaceutical enteric coated pellet formulation comprising dabigatran etexilate free base as an active agent and at least one pharmaceutically acceptable excipient. The present invention further relates to a highly soluble and stable formulation comprising dabigatran etexilate free base as an active agent.

### Background of the Invention

Dabigatran etexilate (Formula 1), which is already known from WO 98/37075, is a direct thrombin inhibitor indicated to reduce the risk of stroke and systemic embolism in patients with non-valcular atrial fibrilation.

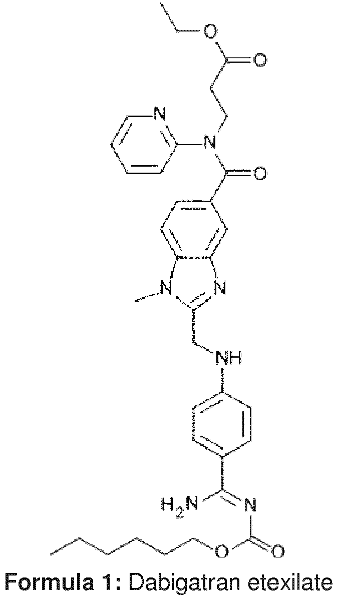

The methane sulphonic acid addition salt of dabigatran etexilate, which is commercially available under the trade name PRADAXA®, is disclosed in EP1870100, wherein also disclosed, pellet formulation of dabigatran etexilate methanesulphonate. This formulation is formulated with a core material consisting of organic acid and an active layer which encloses the core.

Apart from the methanesulfonate salt of dabigatran etexilate, other acid addition salts of the compound are provided in prior art. For exemple, WO2012/077136 is directed to the oxalate salt of dabigatran etexilate and besides, its hydrochloride salt is identified in EP1877395. These various form of dabigatran etexilate are prepared to facilitate the development of pharmaceutical formulation. An active agent should meet some physicochemical requirements in order to be capable of being used in pharmaceutical formulations. These requirements considerably depend on the physicochemical properties of the active agent used in pharmaceutical formulation.

These various dabigatran etexilate salts disclosed in prior art, were compared for their physicochemical properties like water solubility, stability, and shelf-life which are important for the development of pharmaceutical formulations. Also, it is known in the prior art that dabigatran etexilate is a weakly basic compound and therefore it has high solubility in acidic media.

Absorption of the active substances generally occurs in the small intestine due to a large surface area and the slow peristaltic movements of the small intestine that has a basic media. However, absorption from the stomach that has an acidic media is negligibly low due to the fast peristaltic movements and the high the surface area of the stomach. Thus the dissolution of the active substances in the small intestine, a basic media, is important. Dabigatran, is weakly basic, should be dissolved in basic environments and the dissolution of dabigatran should be pH independent.

However, there is still a need for oral pharmaceutically formulations comprising dabigatran etexilate that has pH independent release.

### Object of the Invention

The main object of the present invention is to use of dabigatran etexilate free base in the preparation of a medicament to reduce the risk of stroke and systemic embolism in patients with non-valcular atrial fibrilation.

Another object of the present invention is to obtain a stable and highly soluble pharmaceutical formulations comprising dabigatran etexilate free base as an active agent and at least one pharmaceutically acceptable excipient.

Another object of the present invention is to provide a pharmaceutical formulation comprising dabigatran etexilate free base that has pH-independent release without a dose-dumping.

the purity degree of dabigatran etexilate free base render more stable the pharmaceutical formulation during production and storage.

Another object of the present invention is to provide stable pharmaceutical products during shelf-life, wherein the products comprising dabigatran etexilate free base with a reduced moisture content.

### Detailed Description of the Invention

The present invention is related to novel enteric coated pellet formulations comprising dabigatran etexilate free base as active agent.

As used herein, the term "dabigatran etexilate free base" refers to dabigatran etexilate which is free from other forms of the active moiety, especially acid addition salts.

The dabigatran etexilate free base of the present invention is formed as a solid form which is easily handled and particularly well suited to the pharmaceutical formulation. It has been surprisingly discovered that the dabigatran etexilate free base has physicochemical properties (e.g. purity, non-hygroscopicity, solubility) which are more suitable to develop a pharmaceutical formulation, than other forms of dabigatran etexilate, e.g. salts. According to present invention, it has also been discovered that the pharmaceutical formulation has improved physicochemical properties such as stability, dissolution, storage and flowability, compared to pharmaceutical formulations described in prior art.

According to another embodiment of the present invention, said oral solid pharmaceutical formulation is formulated as enteric coated pellet. Pellets provide a reduction in the dosage regimen and gastrointestinal irritation moreover controlling the drug release and increasing the absorption of the active ingredient. Also one of the advantageous properties of the pellet formulations is being good candidates for the delivery of the drug substances because the pellet formulations minimize the dose dumping effect. The reproducibility of the pellet formulations is also much better than the reproducibility of the single-unit dosage forms. They are suitable systems for film coating with respect to the low surface area-volume ratios. Also, resistance to external factors such as moisture, air and light are the most advantageous properties of these dosage forms. Due to the mentioned reasons, the preferred dosage form is enteric coated pellet.

According to an embodiment of the present invention, using methacrylic acid - ethyl acrylate copolymer (1:1) as an enteric coating agent enhances an increase in the solubility of the active agent in the said formulation. Methacrylic acid - ethyl acrylate copolymer (1:1), an enteric polymer, interacts with the cationic groups of dabigatran etexilate and increases the solubility of dabigatran etexilate. Methacrylic acid - ethyl acrylate copolymer (1:1) is an important factor which controls the solubility of formulations of weakly basic active agents when compared to nonionic coating such as hydroxypropyl methylcellulose (HPMC). In addition, methacrylic acid - ethyl acrylate copolymer (1:1) providing effective and stable coatings is a suitable coating agent for weakly basic active agents such as dagibatran etexilate. In the prior art studies, it is found that using polyinoinic polymers such as methacrylic acid - ethyl acrylate copolymer (1:1) resulted in reduced water uptake and matrix permeability. Thus, stable formulations over the shelf life are obtained by minimizing the penetration of moisture and air into the pellets. Also, methacrylic acid - ethyl acrylate copolymer (1:1) increases the absorption of dabigatran etexilate by providing dissolution of dabigatran etexilate in the upper parts of the small intestine rather than the stomach. The amount of methacrylic acid- ethacrylate copolymer (1:1) is in the range of 0.1 to 50.0%, preferably in the range of of 0.5 to 40.0% by total weight of the formulation.

According to another embodiment of the present invention, it has been observed that adding appropriate buffering agents to the matrix core and coating the dosage form with appropriate coating agents are enough for increasing the solubility of dabigatran etexilate which is a weakly basic compound and for a pH-independent dissolution of dabigatran etexilate. It has been surprisingly discovered that the use of fumaric acid as a buffer agent is effective in increasing the solubility of the active ingredient. The solubility of fumaric acid is lower than that of the other buffering agents. Accordingly, fumaric acid keeps the pH of the micro-environment low, for a long time whilst remain undissolved in tablets or capsules and increases the solubility of the active agent. Also, fumaric acid aids the spheronisation of the pellets due to lubricant property of fumaric acid and contributes the production of fine pellets. By means of fumaric acid, the surface area of the obtained fine pellets is increased and as a result the solubility of the formulation is increased. The amount of fumaric acid is in the range of 0.1 to 50.0%, preferably in the range of of 0.5 to 40.0% by total weight of the formulation.

According to another embodiment of the present invention, in said formulation setting the weight ratio of fumaric acid to methacrylic acid - ethyl acrylate copolymer (1:1) between 80:1 and 1:80 exhibited a synergistic effect on enhancing the stability and solubility of the tablet.

As used herein, the term "methacrylic acid - ethyl acrylate copolymer (1:1)" refers to an anionic copolymer comprising methacrylic acid and ethyl acrylate. The ratio of the carboxylic groups to ester groups in this anionic polymer is about 1:1. The polymer dry weight content of this polymer is between 90.0% to 99.0%, preferably 93.0% to 97.0, more preferably 95.0% based on the total weight of the polymer.

According to an embodiment of the present invention, in said formulation using polyvinylpyrrolidone as a binder increases the stability of the formulation by increasing the viscosity. Polyvinylpyrrolidone increases the solubility of the formulation due to its disintegrant property of polyvinylpyrrolidone. Polyvinylpyrrolidone, is a good binder, aids to the pelletizing process by holding together active agents and excipients. Compared to using non-soluble binders such as gum arabic in the prior art, using PVP is advantagous for the formulations comprising active agents having solubility problems due to high solubility of PVP in water and other polar solvents. Polyvinylpyrrolidone in an amount of 0.05 to 35.00%, preferably 0.1 to 25.00% by weight of total formulation contributes to increasing the stability, the solubility and the shelf life of the formulation.

According to another embodiment of the present invention, besides dabigatran etexilate, methacrylic acid - ethyl acrylate copolymer (1:1), fumaric acid, and polyvinylpyrrolidone, the said formulation further comprises at least one pharmaceutically acceptable excipient selected from the group consisting of diluents, controlled release agents, lubricants, and glidants.

Suitable diluents may include but not limited to sugars, microcrystalline cellulose, mannitol, spray-dried mannitol, lactose, starch, dextrose, sucrose, fructose, maltose, sorbitol, xylitol, inositol, kaolin, inorganic salts, calcium salts, polysaccharides, dibasic calcium phosphate, sodium chloride, dextrates, lactitol, maltodextrin, sucrose-maltodextrin mixture, trehalose, sodium carbonate, sodium bicarbonate, calcium carbonate and mixtures thereof.

The enretric coated pellets according to the present invention in addition to methacrylic acid- ethacrylate copolymer (1:1) may be coated with polyvinil acetate, cellulose acetate phthalate, ammonium methacrylate copolymers, cellulose acetate trimellitate, hydroxypropyl methylcellulose phthalate, polyvinyl acetate phthalate, hydroxypropyl methylcellulose acetate succinate, hydrogels, carboxyvinyl polymer, sodium alginate, sodium carmellose, calcium carmellose, polyvinyl alcohol, polyvinyl alcohol-polyethylene glycol mixtures, gelatine, starch, polyvinylpyrrolidone, microcrystalline cellulose, collagen, hydroxypropyl methylcellulose, hydroxyethyl cellulose, hydroxypropyl cellulose, ethyl cellulose, carboxymethyl cellulose, carnauba wax, pectin, carbomer, poloxamer, polyachrylamide, aluminium hydroxide, bentonit, laponit, setostearyl alcohol, polyoxyethylen-alkyl ethers, hyaluronic acid, guar gum, cocoa oil, paraffin, hydrogenated vegetable oil, cetyl alcohol, stearyl alcohol, stearic acid, xanthane gum or mixtures thereof.

Suitable lubricants may include but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate and mixtures thereof.

Suitable glidants may include but not limited to colloidal silicon dioxide, silicon dioxide, talc, aluminium silicate, silica and mixtures thereof.

According to an embodiment of the present invention, pharmaceutical formulations of the present invention may be prepared by conventional technology well known to those skilled in the art such as extrusion / spheronisation, spray drying and the like. The obtained pellets have at least one coating due to using methacrylic acid- ethacrylate copolymer (1:1).

### Examples:

### Example 1:

| ***Content*** | ***Amount (%)*** |
|---|---|
| Dabigatran etexilate | 15.0 - 60.0 |
| Dibasic calcium phosphate | 5.0 - 40.0 |
| Fumaric acid | 0.5 - 40.0 |
| Polyvinylpyrrolidone (PVP) | 0.1 - 25.0 |
| Methacrylic acid- ethacrylate copolymer (1:1) | 0.5 - 40.0 |
| Silicon dioxide | 0.1 - 0.2 |
| Magnesium stearate | 0.25 - 2.0 |

### Production method:

Dabigatran etexilate, dibasic calcium phosphate, and fumaric acid are mixed and this mixture granulated with hydroalcoholic/ alcoholic PVP solution. The granules that are passed through extruder are exposed to spheronization process to obtain pellets. The obtained pellets are coated with methacrylic acid- ethacrylate copolymer (1:1) solution. The coated pellets are mixed with silicon dioxide and then they are mixed with magnesium stearate for a short time. The obtained pellets are filled to capsules.

### Example 2:

| ***Content*** | ***Amount (%)*** |
|---|---|
| Dabigatran etexilate | 15.0 - 60.0 |
| Fumaric acid | 0.5 - 40.0 |
| Polyvinylpyrrolidone (PVP) | 0.1 - 25.0 |
| Methacrylic acid- ethacrylate copolymer (1:1) | 0.5 - 40.0 |
| Silicon dioxide | 0.1 - 0.2 |
| Magnesium stearate | 0.25 - 2.0 |

### Production method:

The hydroalcoholic/ alcoholic solution that is obtained as a result of mixing with dabigatran etexilate, fumaric acid, and PVP, is pelletized in the spray drier. The obtained pellets are coated with methacrylic acid- ethacrylate copolymer (1:1) solution. The coated pellets are mixed with silicon dioxide and then they are mixed with magnesium stearate for a short time. The obtained pellets are filled to capsules.

### Example 3:

| ***Content*** | ***Amount (%)*** |
|---|---|
| Dabigatran etexilate | 15.0 - 60.0 |
| Fumaric acid | 0.5 - 40.0 |
| Polyvinylpyrrolidone (PVP) | 0.1 - 25.0 |
| Sugar pellets | 5.0 - 90.0 |
| Methacrylic acid- ethacrylate copolymer (1:1) | 0.5 - 40.0 |
| Polyvinyl acetate | 5.0 - 40.0 |
| Silicon dioxide | 0.1 - 0.2 |
| Magnesium stearate | 0.25 - 2.0 |

### Production method:

The hydroalcoholic/ alcoholic solution/ dispersion is prepared by mixing with dabigatran etexilate, fumaric acid, and PVP. The sugar pellets are coated with this solution/ dispersion. Then the obtained pellets are coated with methacrylic acid- ethacrylate copolymer (1:1) solution, then the coated pellets are coated with polyvinyl acetate solution. The coated pellets are mixed with silicon dioxide and then they are mixed with magnesium stearate for a short time. The obtained pellets are filled to capsules.

## Claims

1. An enteric coated pellet formulation, comprising dabigatran etexilate free base as an active agent.

2. The pharmaceutical formulation according to claim 1, further comprising methacrylic acid-ethacrylate copolymer (1:1) as an enteric coating agent.

3. The pharmaceutical formulation according to claim 2, wherein the amount of methacrylic acid- ethacrylate copolymer (1:1) is in the range of 0.1 to 50.0% by total weight of the formulation.

4. The pharmaceutical formulation according to claim 3, wherein the amount of methacrylic acid- ethacrylate copolymer (1:1) is in the range of 0.5 to 40.0% by total weight of the formulation.

5. The pharmaceutical formulation according to any of the preceding claims, further comprising fumaric acid as a buffering agent.

6. The pharmaceutical formulation according to claim 5, wherein the amount of fumaric acid is in the range of 0.1 to 50.0% by total weight of the formulation.

7. The pharmaceutical formulation according to claim 6, wherein the amount of fumaric acid is in the range of 0.5 to 40.0% by total weight of the formulation.

8. The pharmaceutical formulation according to claims 2- 7, wherein the weight ratio of fumaric acid to methacrylic acid- ethacrylate copolymer (1:1) is between 80:1 and 1:80 in the formulation.

9. The pharmaceutical formulation according to any of the preceding claims, further comprises polyvinylpyrrolidone as a binder.

10. The pharmaceutical formulation according to claim 9, wherein the amount of polyvinylpyrrolidone is in the range of 0.05 to 35.0% by total weight of the formulation.

11. The pharmaceutical formulation according to claim 10, wherein the amount of polyvinylpyrrolidone is in the range of 0.1 to 25.0% by total weight of the formulation.

## Patentansprüche

1. Magensaftresistente Pelletformulierung, aufweisend eine dabigatranetexilatfreie Base als Wirkstoff.

2. Pharmazeutische Formulierung nach Anspruch 1, ferner aufweisend Methacrylsäureethacrylatcopolymer (1:1) als ein enterisches Beschichtungsmittel.

3. Pharmazeutische Formulierung nach Anspruch 2, wobei die Menge an Methacrylsäureethacrylatcopolymer (1:1) 0,1 bis 50,0% des Gesamtgewichts der Formulierung beträgt.

4. Pharmazeutische Formulierung nach Anspruch 3, wobei die Menge an Methacrylsäureethacrylatcopolymer (1:1) 0,5 bis 40.0% des Gesamtgewichts der Formulierung beträgt.

5. Pharmazeutische Formulierung nach einem der vorgenannten Ansprüche, ferner aufweisend Fumarsäure als Puffermittel.

6. Pharmazeutische Formulierung nach Anspruch 5, wobei die Menge an Fumarsäure 0,1 bis 50,0% des Gesamtgewichts der Formulierung beträgt.

7. Pharmazeutische Formulierung nach Anspruch 6, wobei die Menge an Fumarsäure 0,5 bis 40,0% des Gesamtgewichts der Formulierung beträgt.

8. Pharmazeutische Formulierung nach Anspruch 2-7, wobei das Gewichtsverhältnis der Fumarsäure zu dem Methacrylsäureethacrylatcopolymer (1:1) zwischen 80:1 und 1:80 in der Formulierung beträgt.

9. Pharmazeutische Formulierung nach einem der vorgenannten Ansprüche, ferner aufweisend Polyvinylpyrrolidon als Bindemittel.

10. Pharmazeutische Formulierung nach Anspruch 9, wobei die Menge an Polyvinylpyrrolidon 0,05 bis 35,0% des Gesamtgewichts der Formulierung beträgt.

11. Pharmazeutische Formulierung nach Anspruch 10, wobei die Menge an Polyvinylpyrrolidon 0,1 bis 25,0% des Gesamtgewichts der Formulierung beträgt.

## Revendications

1. Formulation de pastille à enrobage entérique, comprenant du dabigatran étexilate base libre comme principe actif.

2. Formulation pharmaceutique selon la revendication 1, comprenant en outre un copolymère acide méthacrylique - éthacrylate (1:1) comme agent d'enrobage entérique.

3. Formulation pharmaceutique selon la revendication 2, dans laquelle la quantité de copolymère acide méthacrylique - éthacrylate (1:1) se situe dans la plage de 0,1 à 50,0 % en poids total de la formulation.

4. Formulation pharmaceutique selon la revendication 3, dans laquelle la quantité de copolymère acide méthacrylique - éthacrylate (1:1) se situe dans la plage de 0,5 à 40,0 % en poids total de la formulation.

5. Formulation pharmaceutique selon l'une quelconque des revendications précédentes, comprenant en outre de l'acide fumarique comme agent tampon.

6. Formulation pharmaceutique selon la revendication 5, dans laquelle la quantité d'acide fumarique se situe dans la plage de 0,1 à 50,0 % en poids total de la formulation.

7. Formulation pharmaceutique selon la revendication 6, dans laquelle la quantité d'acide fumarique se situe dans la plage de 0,5 à 40,0 % en poids total de la formulation.

8. Formulation pharmaceutique selon les revendications 2 à 7, dans laquelle le rapport en poids de l'acide fumarique au copolymère acide méthacrylique - éthacrylate (1:1) est compris entre 80:1 et 1:80 dans la formulation.

9. Formulation pharmaceutique selon l'une quelconque des revendications précédentes, comprenant en outre de la polyvinylpyrrolidone comme liant.

10. Formulation pharmaceutique selon la revendication 9, dans laquelle la quantité de polyvinylpyrrolidone se situe dans la plage de 0,05 à 35,0 % en poids total de la formulation.

11. Formulation pharmaceutique selon la revendication 10, dans laquelle la quantité de polyvinylpyrrolidone se situe dans la plage de 0,1 à 25,0 % en poids total de la formulation.
